# EUROPÄISCHE PATENTSCHRIFT

(11) **EP 2 229 645 B1**
(45) Veröffentlichungstag und Bekanntmachung des Hinweises auf die Patenterteilung: **03.07.2019**
(21) Anmeldenummer: 08869773.5
(22) Anmeldetag: 15.12.2008
(51) Int. Cl.: G16H 50/50

(54) **VERFAHREN ZUR FEHLERERKENNUNG IN EINEM STEUERUNGSSYSTEM EINES MEDIZINISCHEN BEHANDLUNGS- UND/ODER DIAGNOSEGERÄTS**
METHOD FOR ERROR RECOGNITION IN A CONTROL SYSTEM OF A MEDICAL TREATMENT AND/OR DIAGNOSIS DEVICE
PROCÉDÉ DE DÉTECTION D'ERREURS DANS UN SYSTÈME DE COMMANDE D'UN APPAREIL DE DIAGNOSTIC ET/OU DE TRAITEMENT MÉDICAL

(30) Priorität: 07.01.2008 DE 102008003440
(43) Veröffentlichungstag der Anmeldung: 22.09.2010
(73) Patentinhaber: KUKA Deutschland GmbH, 86165 Augsburg (DE)
(72) Erfinder: ENGELMANN, Till, 91052 Erlangen (DE); MÜHLHÄUSSER, Matthias, 91074 Herzogenaurach (DE); BEWIG, Lorenz, 85356 Freising (DE); HAGENAUER, Andreas, 86316 Friedberg (DE); HASENZAHL, Torsten, 89407 Dillingen (DE); JACOB, Dirk, 86399 Bobingen (DE); ORTMAIER, Tobias, 30966 Hemmingen (DE); TSCHARNUTER, Dietmar, 86316 Friedberg (DE)
(86) Internationale Anmeldenummer: PCT/EP2008/067496
(87) Internationale Veröffentlichungsnummer: WO 2009/087017

(56) Entgegenhaltungen:
- WO-A1-2004/026395
- JP-A- 3 071 983
- JP-A- 8 141 950
- US-A1- 2007 078 565

## Beschreibung

Die vorliegende Erfindung betrifft ein Verfahren zur Fehlererkennung in einem Steuerungssystem eines medizinischen Behandlungs- und/oder Diagnosegeräts. Die Erfindung betrifft weiterhin ein programmgesteuertes medizinisches Behandlungs- und/oder Diagnosegerät.

Bei vielen Einsatzfällen in der modernen Diagnostik und Therapeutik werden programmgesteuerte Behandlungs- und/oder Diagnosegeräte eingesetzt. Aufgrund der teilweise komplexen Abfolge von Steuerungs- und Bewegungsschritten können diese Geräte auch als Roboter bezeichnet werden, zumal prinzipiell ähnliche Steuerungssysteme zum Einsatz kommen wie bei industriellen Roboteranwendungen. Der Einsatz derartiger Roboter in sicherheitskritischen Applikationen wie in der Medizintechnik stellt sehr hohe Anforderungen an die Systemsicherheit. Es muss gewährleistet werden können, dass eine Fehlfunktion des Roboters erkannt werden kann und dass der Roboter bei einer Fehlfunktion in einen sicheren Zustand gebracht werden kann. Ein sicherer Zustand kann bspw. ein Anhalten des Roboters sein. Zu diesem Zweck muss die korrekte Funktionsweise von kritischen Hard- und Softwarekomponenten im laufenden Betrieb überprüft und eine Fehlfunktion detektiert werden.

Eine Möglichkeit, dies zu erreichen, ist die redundante Ausführung der sicherheitskritischen Komponenten. Es erfolgt weiterhin ein permanenter Vergleich der Ausgangswerte mit Sollwerten, so dass bei einer Abweichung darauf geschlossen werden kann, dass in einer dieser Komponenten ein Fehler aufgetreten ist. Das System kann durch geeignete Maßnahmen in einen sicheren Zustand gebracht werden. Bei diesen Ausführungen von Robotersystemen ist ein sehr hoher Rechenbedarf erforderlich. Insbesondere bei kritischen Softwarekomponenten resultiert ein erhöhter Ressourcenbedarf hinsichtlich Rechenzeit und Speicherplatz. Zudem ist auch die Sicherstellung der Unabhängigkeit der redundanten Komponenten voneinander als nachteilig zu betrachten. Dies gilt gleichermaßen auch für Funktionen, die zu zusätzlichem Hardwareaufwand führen.

Aus der WO2004/026395A1 ist ein Testverfahren eines Steuerungssystem eines implantierbaren medizinischen Geräts, insbesondere eines Herzschrittmachers, bekannt, bei dem die Reaktion des medizinischen Gerätes in einem durch einen Magneten induzierten Testbetrieb für eine vorgegebene Zeitdauer auf die spezifischen Merkmale des medizinischen Geräts angepasst werden kann. In dem offenbarten Testverfahren wird allerdings keine Fehlererkennung durchgeführt.

Der Erfindung liegt die Aufgabe zu Grunde, ein verbessertes Verfahren zur Erkennung und/oder Vermeidung von Fehlern in einem Steuerungssystem zur Verfügung zu stellen, das diese genannten Nachteile vermeidet.

Die Aufgabe der Erfindung wird mit einem Verfahren mit den Merkmalen des Anspruchs 1 gelöst. Hierbei ist ein Verfahren zur Fehlererkennung in einem Steuerungssystem eines medizinischen Behandlungs- und/oder Diagnosegeräts vorgesehen, wobei das Steuerungssystem in einem regulären Betriebsmodus Daten (D) verarbeitet und das Steuerungssystem in einem Testmodus eine sicherheitskritische Komponente des Steuerungssystems mit Testdaten (E) eines Testdatensatzes T(E,A) speist und überprüft. Das Verfahren wird dadurch gekennzeichnet, dass der Testdatensatz T(E,A) als Testdaten Wertepaare enthält, nämlich Eingangswerte (E), die im realen Betrieb bei korrekter Betriebsweise erzeugt wurden, und zugehörige Ausgangswerte (A), die von der sicherheitskritischen Komponente bei korrekter Betriebsweise erzeugt wurden, und dass im Testmodus aus den Eingangswerten (E) mittels der sicherheitskritischen Komponente zugehörige Ausgangswerte (A') erzeugt werden und die erzeugten Ausgangswerte (A') mit den hinterlegten Ausgangswerten (A) des Testdatensatzes T(E,A) verglichen werden, wobei bei einer Abweichung der im Testmodus erzeugten Ausgangswerte (A') von den hinterlegten Ausgangswerten (A) eine Fehlfunktion erkannt wird. Es wird quasi ein Testlauf mit den Testdaten durchgeführt, um die sicherheitskritische Komponente des Steuerungssystems zu überprüfen. Diese Komponente ist insbesondere eine einkanalige nicht redundante Komponente. Das Steuerungssystem umfasst als wesentliche Komponente ein Steuerungsprogramm, das zur Durchführung der hier beschriebenen Schritte ausgebildet ist.

Das Verfahren dient zur Fehlererkennung und/oder -behebung in Steuerungssystemen medizinischer Behandlungs- und/oder Diagnosegeräte, insbesondere von motorischen Verstellsystemen derartiger Behandlungs- oder Diagnosegeräte, wie beispielsweise ein Robotersystem, mit dem eine Behandlungs- oder Diagnoseeinheit oder auch eine Patientenliege in eine definierte Sollposition gebracht wird. Unter regulärer Betriebsmodus wird eine Betriebsart verstanden, bei der eine tatsächliche reale Verstellung des Verstellsystems vorgenommen wird. Unter Testmodus wird eine Betriebsart verstanden, bei der lediglich in einem Speicher hinterlegte Testdaten, die möglichen realen Werten entsprechen, vom Steuerungssystem verarbeitet werden und die dabei erzeugten Ausgangsdaten überprüft werden. Die im Testmodus ermittelten Ausgangsdaten werden mit Sollwerten, die auch als Referenzdaten bezeichnet werden, verglichen. Dies sind insbesondere hinterlegte Testdaten. Bei einer Abweichung der im Testmodus ermittelten Ausgangsdaten von den hinterlegten Testdaten um vorgegebene Werte kann auf diese Weise ein Programm- und/oder Gerätefehler erkannt werden.

Die vorliegende Erfindung zeigt somit eine Möglichkeit, auf eine redundante Ausführung der kritischen Komponenten mit den oben beschriebenen Nachteilen zu vermeiden. Eine Verifikation der korrekten Funktionsweise der Programmabläufe ist zyklisch und/oder azyklisch im laufenden Betrieb oder vor einer Inbetriebnahme möglich.

Der erfindungsgemäße Lösungsansatz erlaubt insbesondere eine Detektierung von zufälligen Fehlern (z.B. Speicherüberlauf, Kippen von Bits etc.).

Der Testdatensatz enthält als Testdaten Wertepaare, nämlich Eingangswerte, die im realen Betrieb als Daten auftreten können, und zugehörige Ausgangswerte als Referenzdaten, die von der sicherheitskritischen Komponente bei korrekter Betriebsweise erzeugt werden. Im Testmodus wird der Komponente jeweils ein Eingangswert eingespeist und der von der Komponente errechnete Ausgangswert wird mit dem jeweiligen im Testdatensatz hinterlegten, zum Eingangswert zugehörigen Ausgangswert verglichen. Es wird daher mit dem Testmodus der reale Betrieb simuliert und bei einer Abweichung der er-rechneten Daten von den hinterlegten zu erwartenden Daten auf einen Fehler geschlossen.

Bei den von der Komponente verarbeiteten Daten handelt es sich zweckdienlicherweise im Betriebsmodus um Positionsdaten eines Verstellsystems, die vom Steuerungssystem aufbereitet werden und insbesondere einem medizinischen Steuerungssystem für einen Soll-Ist-Vergleich übermittelt werden. Es wird daher eine sicherheitskritische Berechnung von Positionsdaten im Testmodus überprüft, um zu vermeiden, dass das medizinische Steuerungssystem von falsch errechneten IST-Daten ausgeht. Dies kann dazu führen, dass das medizinische Steuerungssystem wegen der falschen Ortsinformationen falsche Steuerbefehle an die Robotersteuerung abgibt und eine erforderliche hochgenaue Positionierung beispielsweise des Patienten oder einer Therapie- oder Diagnoseeinheit, wie beispielsweise ein Röntgenstrahler, nicht mehr erfolgen kann. Oder dass eine falsche Positionierung vom medizinischen Steuerungssystem nicht erkannt wird.

Zweckdienlicherweise ist die zu überprüfende sicherheitskritische Komponente ein Aufbereitungsmodul, in dem die eingangsseitigen Positionsdaten einer Koordinatentransformation unterzogen werden. Bei dem Verstellsystem handelt es sich bevorzugt um einen mehrachsigen (Industrie-) Roboter. Dessen Verstellmotoren werden von dem Steuerungssystem, der Robotersteuerung, angesteuert. Die Robotersteuerung rechnet hier üblicherweise mit achsspezifischen Koordinaten des Verstellsystems. Vom medizinischen Steuerungssystem werden demgegenüber regelmäßig kartesische Positionsdaten vorgegeben. Innerhalb der Robotersteuerung wird daher in einer sogenannten Vorwärtstransformation eine Transformation der achsspezifischen Koordinaten in kartesische Koordinaten vorgenommen. Diese Umrechnung erfolgt in der sicherheitskritischen Komponente.

Vorzugsweise wird vor einer ersten Inbetriebnahme des Systems im Betriebsmodus der Testdatensatz für die zu überwachende Komponente generiert. Bevor die sicherheitskritische Funktion mit den "realen" Daten erstmalig aufgerufen wird, wird die korrekte Funktionsweise mittels des Testdatensatzes überprüft. Alternativ oder ergänzend ist zweckdienlicherweise vorgesehen, dass der Testdatensatz während einer korrekten Betriebsweise des Geräts erzeugt wird. Auch besteht die Möglichkeit, korrekte Testdaten oder einen gesamten Testdatensatz aus einer externen Quelle einzulesen.

Zweckdienlicherweise wird der Testmodus ständig wiederkehrend, beispielsweise mehrmals nacheinander und/oder in zyklischen Zeitabständen durchgeführt, so dass eine permanente Überprüfung des Systems erfolgt. Und zwar vorzugsweise so, dass alle relevanten Pfade der sicherheitskritischen Komponente wenigstens einmal durchlaufen werden. Der Testmodus wird vorzugsweise vielfach häufiger als der reale Betriebsmodus durchgeführt.

Bevorzugt wird hierbei der Testmodus unter Anpassung an die zur Verfügung stehende Rechenleistung ausgeführt, beispielsweise zyklisch und/oder azyklisch. Damit wird verhindert, dass der reale Betrieb nicht durch den Testmodus verlangsamt oder gestört wird.

Die mit den Eingangsdaten berechneten Ergebnisse werden mit den gespeicherten Ausgangswerten verglichen. Bei einer Abweichung über einen Schwellwert hinaus wird auf eine Fehlfunktion der kritischen Komponente geschlossen. Wahlweise kann bei einem erkannten Fehler ein Warnsignal erzeugt und/oder das Gerät in einen sicheren Zustand überführt werden. Ggf. kann auch ein weiteres Signal erzeugt werden, das abgespeichert und zur späteren Auswertung der aufgetretenen Fehlerart und/oder Fehlerhäufigkeit verwendet werden kann.

Eine vorteilhafte Variante des Verfahrens ist dadurch gekennzeichnet, dass die Testdaten für den Testmodus definierte fehlerhafte Werte aufweisen. Hierdurch kann auch das Testverfahren selbst einer Überprüfung unterzogen werden, da die Erkennung eines Fehlers ebenfalls überprüft werden kann. Dieser Schritt dient daher zur Überprüfung der Funktionsfähigkeit der Fehlererkennung des Systems.

Die Aufgabe wird erfindungsgemäß weiterhin gelöst durch ein medizinischen Behandlungs-/ oder Diagnosegerät nach Anspruch 11. Die auf das Verfahren gerichteten Vorteile und bevorzugten Varianten sind sinngemäß auch auf das Gerät zu übertragen.

Weitere Merkmale der Erfindung gehen aus der nun folgenden detaillierten Beschreibung einer bevorzugten Ausführungsform der Erfindung hervor, die als nicht einschränkendes Beispiel dient und auf die beigefügten Zeichnungen Bezug nimmt.
FIG 1 zeigt anhand eines schematischen Blockdiagramms den Gesamtablauf einer Steuerung eines medizinischen Diagnose oder Behandlungsgeräts,
FIG 2 zeigt einen Teilbereich eines Steuerungssystems in einer Blockbilddarstellung.

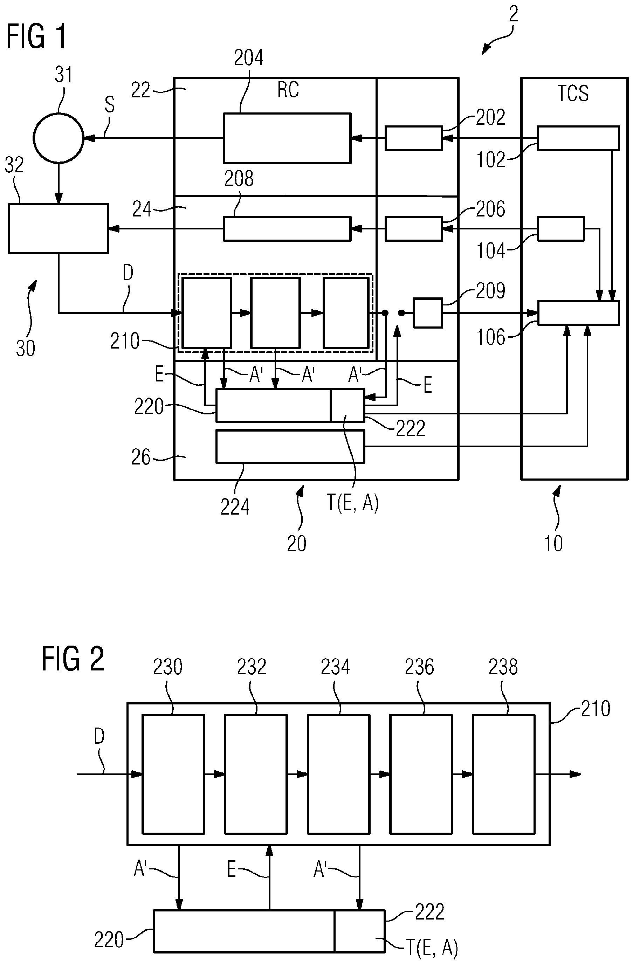

Eine mögliche Ausgestaltung eines Verfahrens zur Fehlererkennung bei einem medizinischen Gerät 2 wird anhand der FIG 1 und 2 illustriert. In einer besonders bevorzugten Ausprägungsform wird das System zur sicheren Verifikation der absolut genauen Position eines absolut genau vermessenen Roboters eingesetzt. In dieser Applikation ist die zu überwachende sicherheitskritische Funktion das auf einer Robotersteuerung gerechnete absolut genaue Robotermodell.

Mit derzeit bekannten Steuerungen kann für hochgenaue Positionsanforderungen die Position einer Maschine bzw. eines Roboters nur durch ein zusätzliches Messsystem verifiziert werden, da nur auf diese Weise zuverlässig überprüft werden kann, ob der Roboter auch exakt die Position innehat, die ihm von der Steuerung vorgegeben worden ist. Bei einer sog. absolutgenauen Maschine kann die angefahrene, absolutgenaue Position nur mit einem zusätzlichen Messsystem wie bspw. einem Lasertracker oder einer Koordinatenmessmaschine verifiziert werden. Mit Hilfe des beschriebenen Verfahrens wird ein sicheres Anfahren einer absolutgenauen Position eines absolutgenauen Roboters ohne zusätzliches Messsystem gewährleistet. Dadurch ist eine erhebliche Kostenreduktion möglich. Eine Möglichkeit einer konkreten Umsetzung wird anhand der Flussdiagramme der Figuren 1 und 2 skizziert.

Das Verfahren zur Verifikation der anzufahrenden Position wird auch als "Gesundheitscheck" bezeichnet, womit die richtige Funktionsweise und somit die "Gesundheit" der zu überwachenden sicherheitskritischen Komponente gemeint ist. Der Gesundheitsscheck bildet daher eine Überwachungsfunktion.

Im Blockdiagramm der FIG 1 verdeutlicht der mit "TCS" (Therapy Control System) bezeichnete rechte Block den gesamten Teil der Steuerung des medizinischen Gerätes 2, nachfolgend als medizinisches Steuerungssystem 10 bezeichnet. Dieses gibt bspw. die Bewegungsdaten für eine Untersuchung im Rahmen einer Computertomografie vor und überprüft diese auch.

Der mit "RC" (Robot Control) bezeichnete größere mittlere Block verdeutlicht den gesamten Bereich der eigentlichen Robotersteuerung und damit den maschinennäheren Bereich, nachfolgend als Steuerungssystem 20 bezeichnet. Mit dieser Steuerung wird das eigentliche Verstellsystem 30 angesteuert, insbesondere Verstellmotoren 31 des Roboters.

Mit Hilfe von vom medizinischen Steuerungssystems 10 vorgegebenen Solldaten 102 werden Fahrbefehle 202 des Steuerungssystems 20 erzeugt, welche nach Durchlaufen einer sog. Bahnplanung 204 zur Erzeugung von Steuerungssignalen S für die Motoren 31 weiterverarbeitet werden. Hierbei werden die üblicherweise als kartesische Koordinaten vorgegebenen Solldaten 102 in einer sogenannten Rückwärtstransformation in achsspezifische Koordinaten des üblicherweise mehrachsigen Roboters transformiert. Dieser Bereich des Steuerungssystems 20 wird auch als regelnder Kanal 22 bezeichnet.

Vom Steuerungssystem 10 erfolgt weiterhin eine Abfrage 104 einer aktuellen, tatsächlichen Ist-Position des Verstellsystems 30, die in einem Überwachungskanal 24 des Steuerungssystems 20 in Zwischenschritten 206, 208 weiterverarbeitet und an ein sicheres Erfassungssystem 32 des Verstellsystems 30 übermittelt wird. Einer jeweiligen Abfrage wird hierbei eine sogenannte Sequenznummer als eindeutige Kennung zugeordnet.

Das sichere Erfassungssystem 32 liefert nach Art einer Antwort auf die Abfrage an das Steuerungssystem 20 insgesamt zahlreiche Daten D über den aktuellen Zustand des Verstellsystems 30, insbesondere die aktuellen Positionen. Dies sind z.B. achsspezifische Positionsdaten der einzelnen Roboterachsen zu den aktuellen IST-Positionen der einzelnen Robotertei-Ie bzw. Roboterachsen, zugehörige kartesische Koordinaten entsprechend einem hinterlegten Standard-Modell sowie auch sogenannte Prüfsummen, anhand derer beispielsweise eine Überprüfung der Konsistenz oder Plausibilität der Daten D durchgeführt werden kann. Die aktuellen Ist-Daten werden mit der zugehörigen Sequenznummer gekennzeichnet.

In einem Aufbereitungsmodul 210 des Überwachungskanals 24 werden allgemein die bereitgestellten Daten rechnerisch aufbereitet und die aufbereiteten Daten werden ggf. über einen Puffer 209 einem Vergleichsmodul 106 des Steuersystems 10 übermittelt. In dem Vergleichsmodul 106 findet ein Vergleich der IST-Daten mit den zuvor vorgegebenen SOLL-Daten statt. Das Aufbereitungsmodul 210 kann Teil einer Robotersteuerung oder eines medizinischen Gerätes, bzw. dessen Komponenten sein.

Diese Aufbereitung der Daten in dem Aufbereitungsmodul 210 ist - da nicht redundant und nur einkanalig ausgebildet - sicherheitskritisch. Zur Überprüfung dieser sicherheitskritischen Aufbereitung wird in einer Verifizierungsebene 26 des Steuerungssystems 20 im Rahmen eines sog. "Gesundheitschecks" in einem Verifizierungsmodul 220 die Funktion des Aufbereitungsmoduls 210 überprüft. Hierzu ist in einem Speicher 222 in einer Tabelle ein Testdatensatz T (E, A) abgelegt. Dieser wird vor Erst-Inbetriebnahme des realen Betriebs generiert und enthält quasi als Testdaten Wertepaare, nämlich die Eingangswerte (E) sowie die zu erwartenden Ausgangswerte (A) für das Aufbereitungsmoduls 210.

Weiterhin ist in der Verifizierungsebene 26 ein Messmodul 224 vorgesehen, welches weitere Kenndaten an das Steuersystem 10 zum SOLL-IST-Vergleich weiterleitet. Diese Kenndaten sind beispielsweise die Motorströme der einzelnen Motoren 31 für die jeweiligen Roboterachsen; Lastdaten, also beispielsweise das Gewicht eines Patienten, der sich auf einer vom Verstellmechanismus bewegten Patientenliege befindet, oder Werkzeugdaten, die beispielsweise Daten über die Durchbiegung, Torsion etc. enthalten.

Die Durchführung eines Testmodus mit Hilfe des Verifizierungsmoduls 220 wird nachfolgend in Verbindung mit anhand der FIG 2 näher erläutert:
Die Aufbereitung der Daten D im Aufbereitungsmodul 210 erfolgt in mehreren Schritten 230-238. Im ersten Schritt 230 erfolgt eine PrüfSummenüberprüfung, um zunächst die Konsistenz der Daten (D) zu prüfen. Anschließend erfolgt im zweiten Schritt 232 die hochgenaue sogenannte Vorwärtstransformation von den achsspezifischen Koordinaten in kartesische Koordinaten. Diese werden in einem anschießenden dritten Schritt 234 einem Plausibilitätscheck durch Vergleich mit den vom sicheren Erfassungssystem 32 mitgelieferten kartesischen Koordinaten unterzogen. Schließlich erfolgt in einem vierten Schritt 236 eine weitere Koordinatentransformation entsprechend einer Norm, auf deren Grundlage das Steuerungssystem 10 arbeitet. Abschließend wird im fünften Schritt 238 noch eine Prüf- oder Checksummenbildung für einen nachfolgenden Konsistenz- oder Plausibilitätscheck vorgenommen, um die Daten sicher zu machen.

Zur Durchführung des Testmodus speist das Verifizierungsmodul 220 das Aufbereitungsmodul 210 mit Eingangswerten E und liest die jeweiligen errechneten Ausgangswerte A' aus und überprüft diese anhand eines Vergleichs mit den im Speicher 222 hinterlegten Ausgangswerten A.

Dieser Testmodus wird bevorzugt vor einem ersten realen Betriebsmodus als auch während des Betriebsmodus oder zwischen aufeinanderfolgenden Betriebsmodi durchgeführt, bei denen eine reale Ansteuerung des Verstellmechanismus 30 erfolgt. Durch den zyklischen oder permanenten Vergleich über das Verifizierungsmodul 220 wird die Funktionsfähigkeit des sicherheitskritischen Aufbereitungsmoduls 210 überwacht. Die Durchführung des Testmodus erfolgt bevorzugt während Zeiten geringer Prozessorauslastung, um eine gegebene Rechenkapazität optimal auszunutzen und den normalen Betriebsmodus nicht zu stören.

Wird vom Verifizierungsmodul 220 ein Fehler erkannt, so wird das gesamte Verstellsystem 30 in einen sicheren Zustand überführt.

## Patentansprüche

1. Verfahren zur Fehlererkennung in einem Steuerungssystem (20) eines medizinischen Behandlungs- und/oder Diagnosegeräts (2), wobei das Steuerungssystem (20) in einem regulären Betriebsmodus Daten (D) verarbeitet und das Steuerungssystem (20) in einem Testmodus eine sicherheitskritische Komponente (210) des Steuerungssystems (20) mit Testdaten (E) eines Testdatensatzes T(E,A) speist und überprüft, **dadurch gekennzeichnet, dass** der Testdatensatz T(E,A) als Testdaten Wertepaare enthält, nämlich Eingangswerte (E), die im realen Betrieb bei korrekter Betriebsweise erzeugt wurden, und zugehörige Ausgangswerte (A), die von der sicherheitskritischen Komponente (210) bei korrekter Betriebsweise erzeugt wurden, und dass im Testmodus aus den Eingangswerten (E) mittels der sicherheitskritischen Komponente (210) zugehörige Ausgangswerte (A') erzeugt werden und die erzeugten Ausgangswerte (A') mit den hinterlegten Ausgangswerten (A) des Testdatensatzes T(E,A) verglichen werden, wobei bei einer Abweichung der im Testmodus erzeugten Ausgangswerte (A') von den hinterlegten Ausgangswerten (A) eine Fehlfunktion erkannt wird.

2. Verfahren nach Anspruch 1, bei dem es sich bei den Daten im Betriebsmodus um reale Positionsdaten (D) eines Verstellsystems (30) handelt, die vom Steuerungssystem (20) aufbereitet und einem medizinischen Steuerungssystem (10) übermittelt werden, wo die aufbereiteten Positionsdaten einem SOLL-IST-Vergleich unterzogen werden.

3. Verfahren nach Anspruch 2, bei dem die sicherheitskritische Komponente ein Aufbereitungsmodul (210) ist, in dem die eingangsseitigen Positionsdaten (D) einer Koordinatentransformation unterzogen werden.

4. Verfahren nach einem der vorhergehenden Ansprüche, bei dem der Testdatensatz T(E,A) während einer korrekten Betriebsweise des Gerätes (2) erzeugt oder aus einer externen Quelle bereitgestellt wird.

5. Verfahren nach einem der vorhergehenden Ansprüche, bei dem der Testmodus ständig wiederkehrend ausgeführt wird.

6. Verfahren nach Anspruch 5, bei dem der Testmodus zyklisch und/oder azyklisch ausgeführt wird.

7. Verfahren nach einem der vorhergehenden Ansprüche, bei dem bei einer erkannten Fehlfunktion ein Warnsignal erzeugt und/oder das Gerät in einen sicheren Zustand überführt wird.

8. Verfahren nach einem der vorhergehenden Ansprüche, bei dem die Testdaten (E,A) zur Überprüfung der Funktionsfähigkeit der Fehlererkennung für den Testmodus definierte fehlerhafte Werte aufweisen.

9. Programmgesteuertes Medizinisches Behandlungs- und/oder Diagnosegerät (2), das zur Durchführung des Verfahrens zur Fehlererkennung gemäß einem der vorhergehenden Ansprüche ausgebildet ist.

## Claims

1. Method for error recognition in a control system (20) of a medical treatment and/or diagnostic device (2), wherein the control system (20) processes data (D) in a regular operating mode and the control system (20) feeds test data (E) of a test dataset T(E,A) to and checks a safety-critical component (210) of the control system (20) in a test mode, **characterized in that** the test dataset T(E,A) contains pairs of values as test data, namely input values (E) that were generated during real operation during the correct mode of operation, and associated output values (A) that were generated by the safety-critical component (210) during the correct mode of operation, and **in that**, in the test mode, associated output values (A') are generated from the input values (E) by way of the safety-critical component (210) and the generated output values (A') are compared with the stored output values (A) of the test dataset T(E,A), wherein a malfunction is recognized if the output values (A') generated in the test mode deviate from the stored output values (A).

2. Method according to Claim 1, wherein the data in the operating mode are real position data (D) of an adjustment system (30) that are prepared by the control system (20) and transmitted to a medical control system (10) where the prepared position data are subjected to a target-actual comparison.

3. Method according to Claim 2, wherein the safety-critical component is a preparation module (210) in which the input-side position data (D) are subjected to a coordinate transformation.

4. Method according to one of the preceding claims, wherein the test dataset T(E,A) is generated during a correct mode of operation of the device (2) or is provided from an external source.

5. Method according to one of the preceding claims, wherein the test mode is executed in a constantly repeating manner.

6. Method according to Claim 5, wherein the test mode is executed cyclically and/or acyclically.

7. Method according to one of the preceding claims, wherein, if a malfunction is recognized, a warning signal is generated and/or the device is transferred into a safe state.

8. Method according to one of the preceding claims, wherein the test data (E,A) for checking the functionality of the error recognition for the test mode have defined incorrect values.

9. Program-controlled medical treatment and/or diagnostic device (2) designed to perform the error recognition method according to one of the preceding claims.

## Revendications

1. Procédé de reconnaissance des défauts dans un système de commande (20) d'un appareil de traitement et/ou de diagnostic médical (2), le système de commande (20), dans un mode de fonctionnement normal, traitant des données (D) et le système de commande (20), dans un mode de test, alimentant un composant essentiel à la sécurité (210) du système de commande (20) avec des données de test (E) d'un jeu de données de test T(E, A) et le contrôlant, **caractérisé en ce que** le jeu de données de test T(E, A) contient des paires de valeurs en tant que données de test, à savoir des valeurs d'entrée (E), qui ont été générées en fonctionnement réel lors d'un régime de fonctionnement correct, et des valeurs de sortie (A) associées, qui ont été générées par le composant essentiel à la sécurité (210) lors d'un régime de fonctionnement correct, et **en ce que** dans le mode de test, des valeurs de sortie (A') associées sont générées au moyen du composant essentiel à la sécurité (210) à partir des valeurs d'entrée (E) et les valeurs de sortie (A') générées sont comparées avec les valeurs de sortie (A) stockées du jeu de données de test T(E, A), un défaut de fonctionnement étant reconnu dans le cas d'un écart entre les valeurs de sortie (A') générées dans le mode de test et les valeurs de sortie (A) stockées.

2. Procédé selon la revendication 1, selon lequel les données dans le mode de fonctionnement sont des données de position réelles (D) d'un système de positionnement (30), lesquelles sont conditionnées par le système de commande (20) et sont communiquées à un système de commande médical (10) où les données de position conditionnées sont soumises à une comparaison consigne-réel.

3. Procédé selon la revendication 2, selon lequel le composant essentiel à la sécurité est un module de conditionnement (210) dans lequel les données de position (D) côté entrée sont soumises à une transformation de coordonnées.

4. Procédé selon l'une des revendications précédentes, selon lequel le jeu de données de test T(E, A) est généré pendant un régime de fonctionnement correct de l'appareil (2) ou mis à disposition depuis une source externe.

5. Procédé selon l'une des revendications précédentes, selon lequel l'exécution du mode de test est répétée continuellement.

6. Procédé selon la revendication 5, selon lequel le mode de test est exécuté de manière cyclique et/ou acyclique.

7. Procédé selon l'une des revendications précédentes, selon lequel, lorsqu'un défaut de fonctionnement est reconnu, un signal d'alerte est généré et/ou l'appareil est transféré dans un état sécurisé.

8. Procédé selon l'une des revendications précédentes, selon lequel les données de test T(E, A) possèdent des valeurs erronées définies en vue de contrôler l'aptitude fonctionnelle de la reconnaissance de défaut.

9. Appareil de traitement et/ou de diagnostic médical (2) programmable, lequel est configuré pour mettre en oeuvre le procédé de reconnaissance des défauts selon l'une des revendications précédentes.
